(19) Europäisches Patentamt
European Patent Office
Office européen des brevets

(11) Numéro de publication: 0 277 061 A2

(12) **DEMANDE DE BREVET EUROPEEN**

(21) Numéro de dépôt: 88400087.8

(22) Date de dépôt: 18.01.88

(51) Int. Cl.4: C 07 F 5/00

(30) Priorité: 22.01.87 FR 8700835
09.12.87 FR 8717270

(43) Date de publication de la demande:
03.08.88 Bulletin 88/31

(84) Etats contractants désignés: BE DE GB IT NL

(71) Demandeur: INSTITUT FRANCAIS DU PETROLE
4, Avenue de Bois-Préau
F-92502 Rueil-Malmaison (FR)

(72) Inventeur: Chauvin, Yves
64, avenue du Général Leclerc
F-78230 Le Pecq (FR)

Commereuc, Dominique
32, rue Abel Vacher
F-92190 Meudon (FR)

Hugues, François
2, Av. Félix Faure
F-92000 Nanterre (FR)

Olivier, Hélène
72, avenue de la République
F-92500 Rueil Malmaison (FR)

Saussine, Lucien
2, Place des Frères Tissandier
F-78290 Croissy Sur Seine (FR)

(54) **Nouveau procédé d'obtention d'organolanthanides, nouveaux composés ainsi obtenus et leur utilisation.**

(57) Nouveau procédé d'obtention d'organolanthanides par réaction d'un métal de terre rare sur un composé insaturé en milieu éther. Nouveaux composés ainsi obtenus. Utilisation de ces composés en synthèse organique et pour la catalyse.

EP 0 277 061 A2

## Description

La présente invention a pour objet un nouveau procédé d'obtention d'organolanthanides comportant au moins une liaison métal-carbone, les nouveaux composée obtenus par cette voie et leurs dérivés, ainsi que l'utilisation de ces composés pour la synthèse et la catalyse.

Les différentes voies actuellement connues d'obtention d'organo lanthanides divalents mettent en oeuvre :
- soit la réaction d'un composé organométallique de la série principale, tel qu'un organosodique, un organolithien, un organomagnésien ou un organoaluminique, sur un sel bivalent de lanthanide tel que chlorure, bromure ou carboxylate ;
- soit une transmétallation entre le métal, l'ytterbium par exemple, et un organomercurique, tel le dicyclopentadiènylmercure ;
- soit la dissolution du métal, tel que l'europium ou l'ytterbium par exemple, dans l'ammoniac liquide puis réaction avec un hydrocarbure comportant un hydrogène acide ($pKa < 20$) comme le cyclopentadiène ;
- soit la co-condensation, à basse température, par exemple - 196°C, de vapeur de métal et d'un substrat insaturé.

Les deux premières méthodes nécessitent l'utilisation d'un organométallique obtenu par d'autres voies puis la séparation des sous-produits ; les deux dernières techniques sont complexes, la méthode à l'ammoniac liquide conduisant à des sous-produits amminés et la méthode de vaporisation étant limitée par les quantités de métal qu'on peut pratiquement vaporiser. D'autre part chacune d'entre elles ne conduit qu'à des composés qui lui sont spécifiques.

Il a maintenant été trouvé qu'un organolanthanide comportant au moins une liaison métal-carbone peut être obtenu par réaction directe d'un métal de terre rare (ou lanthanide) avec un composé organique cyclique ou acyclique comportant au moins deux insaturations appartenant aux types C = C, C = O, et/ou C = N. On opère en milieu éther, en absence d'oxygène et de tout composé à proton actif, tel que, par exemple, eau, alcool, ammoniac. Par exemple, avec un hydrocarbure insaturé, on peut former un composé de stoechiométrie hydrocarbure (molécule) : métal (atome) de 1:1. Par insaturation C = C on entend l'insaturation éthylénique et l'insaturation aromatique.

Les hydrocarbures insaturés qui peuvent être mis en oeuvre selon l'invention sont, par exemple, les polyoléfines conjuguées, notamment les di-, tri-, tétraoléfines conjugées, et les hydrocarbures aromatiques comportant plusieurs cycles, de préférence condensés.

Les di-, tri-, tétraoléfines conjugées sont particulièrement celles définies par les formules génériques :

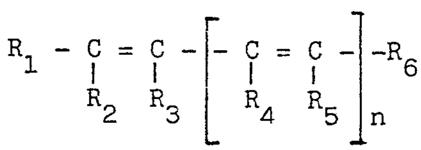

et

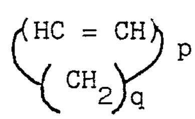

(1) (2)

dans lesquelles n prend des valeurs entières égales à 1,2 ou 3, p de valeurs entières égales à 2,3 ou 4, q étant égal à zéro ou aux valeurs entières de 1 à 5, la somme $p+q$ étant au moins égale à 4.

Dans la formule (1) $R_1$, $R_2$, $R_3$, $R_4$, $R_5$ et $R_6$, identiques ou différents, sont choisis dans le groupe constitué de l'atome d'hydrogène et des restes hydrocarbyles de 1 à 20 atomes de carbone, par exemple alkyl, alkényl, cycloalkyl, cycloalkényl, aryl, aralkyl, etc... Il est préférable cependantq que $R_1$ ou $R_2$ et $R_5$ ou $R_6$ soient l'hydrogène, de sorte que les atomes de carbone terminaux ne soient pas complètement substitués.

Le procédé est applicable aux multioléfines de $C_4$ à $C_{40}$, les exemples de dioléfines étant le butadiène, l'isoprène, le pipérylène, le diméthyl-2,3 butadiène, le phényl-1 butadiène, le diphényl-1,4 butadiène, le cyclooctadiène-1,3 ; parmi les trioléfines : l'hexatriène 1, 3, 5, l'octatriène-2, 4, 6, le cycloheptatriène-1, 3,5 ; un exemple de tétraoléfine étant le cyclooctatétraène.

Des exemples d'hydrocarbures aromatiques condensés utilisables selon l'invention et renfermant de préférence de 2 à 5 cycles, sont le naphtalène, l'acénaphtène, l'acénaphtylène, l'anthracène, le diméthyl-9,10 anthracène, le bis-triméthylsilyl-9,10 anthracène, le phénanthrène, le chrysène, le pyrène et le pérylène.

D'autres composés utilisables dans l'invention comprennent les cétones, en particulier les cétones aromatiques et oléfiniques conjuguées, et les esters, en particulier les esters aromatiques et oléfiniques conjugués.

Les cétones dont l'emploi est préféré selon l'invention, sont les monocétones de formule générale R COR[1](3), les dicétones de formule RCO X COR[1] (4) et les quinones de formule générale :

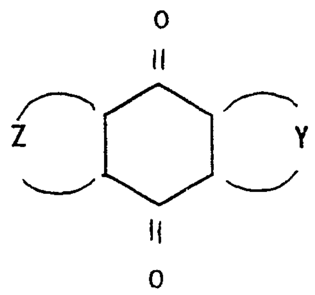

(5)

Les esters préférés ont pour formule générale ArCOOR (6).

Dans ces formules Ar désigne un radical aryl ou alkylaryl comportant de 6 à 20 atomes de carbone, R désigne un reste Ar défini comme ci-dessus ou un reste alkényl ou cycloalkényl comportant de préférence de 2 à 20 atomes de carbone et RI désigne un radical hydrocarbyl, particulièrement un radical alkyl, cycloalkyl, alkényl, cycloalkényl, aryl, alkylaryl ou aralkyl comportant de 1 à 20 atomes de carbone ; X est une liaison simple ou désigne un reste alkylène de 2 à 10 atomes de carbone ; Y est Z sont une liaison simple ou complètent un cycle aromatique, c'est-à-dire ont une formule générale CR = CR-CR = CR où R est un atome d'hydrogène ou un radical alkyle.

A titre d'exemples on peut citer la benzophénone, la chalcone, le benzile, la benzoquinone, la naphtoquinone. L'ester est par exemple le benzoate de phényle ou le benzoate de benzyle.

Les composés possédant une ou plusieurs doubles liaisons carbone-azote, dans un enchaînement acyclique ou cyclique, et en particulier les composés dans lesquels cette liaison est conjuguée avec une ou des doubles liaisons carbone-carbone, carbone-oxygène et/ou carbone-azote et/ou avec un ou des cycles aromatiques, s'additionnent également sur les terres rares en milieu éther. Parmi ces composés on peut citer la quinoléine, l'isoquinoléine, l'acridine, la phénazine, la pyridazine, la phénanthroline, les azocines et les bases de Schiff résultant de l'addition d'une amine sur une cétone ou une aldéhyde telle la benzilidène aniline.

Les métaux susceptibles de réagir avec les hydrocarbures insaturés, les cétones insaturées, les esters insaturés et les composés azotés insaturés appartiennent à la classe des lanthanides, c'est-à-dire le lanthane et les métaux qui, dans le classification périodique des éléments, suivent le lanthane, à savoir le cérium, le praséodyme, le néodyme, le samarium, l'europium, le gadolinium, le terbium, le dysprosium, l'holmium, l'erbium, le thulium, l'ytterbium et le lutétium, mais plus particulièrement le samarium, le néodyme, le cérium, l'europium et l'ytterbium. Ils sont de préférence mis en oeuvre sous la forme de poudres métalliques fines, de copeaux ou de granules, et activés au préalable par de petites quantités, représentant par exemple de 0,1 à 10 % molaire par rapport au métal, d'halogénures d'hydrocarbyle, notamment d'halogénures d'alkyle, d'alkényle, de cycloalkyle, d'aryle ou d'arylalkyle tels que l'iodure de méthyle, le bromure de méthyle, le diiodo-1,2 éthane, le chlorure de benzyle, ou encore d'halogène, de préférence d'iode moléculaire, d'halogénures mercuriques ou d'halogénures de métaux des terres rares tels que, par exemple les iodures.

On peut aussi activer la surface métallique par trituration ou encore par utilisation d'ultrasons.

Le milieu préféré dans lequel on fait réagir le métal sur le composé insaturé renferme un mono ou un polyéther, cyclique ou acyclique comportant de 2 à 20 atomes de carbone. Parmi les éthers préférés on peut citer le diméthyléther, le tétrahydrofuranne, le diméthyléther de l'éthylène glycol, le diméthyléther du diéthylène glycol, le diméthyléther du triéthylène glycol, les éthers cycliques du glycol éthylénique comportant par exemple 4, 5, 6, 7 ou 8 motifs $-CH_2CH_2O-$. On peut également utiliser des éthers cycliques ou non comportant une ou plusieurs fonctions amines comme la tris (méthoxy-2 éthoxy)-2 ethyl amine ou les cryptants. On peut utiliser ces éthers en mélange avec un ou plusieurs hydrocarbures, aliphatiques, cycloaliphatiques ou aromatiques, par exemple l'heptane ou le toluène, dans des proportions pondérales qui dépendent de l'éther, le rapport éther : hydrocarbures allant par exemple de 0,1 à 10 et plus, les rapports les plus faibles étant utilisés de préférence avec les éthers cycliques du glycol.

Le volume de solvant, ou de mélange de solvants par gramme de métal mis en oeuvre peut aller par exemple de 1 à 100 ml.

Le rapport molaire entre le composé organique insaturé et le métal peut varier dans de grandes limites, par exemple dans le rapport mol.gr. de composé insaturé/at.gr. de métal de 1/20 à 20/1, de préférence 1/5 à 5/1. Si on veut favoriser la conversion du composé organique insaturé, on mettra un excès de métal ; inversement, pour favoriser la conversion du métal, on utilisera un excès de composé organique insaturé.

La température à laquelle se fait la réaction sera comprise de préférence entre -80°C et +100°C, elle peut être constante ou il peut être avantageux de la faire varier au cours de la réaction. Une élévation accélère la réaction mais peut éventuellement défavoriser l'équilibre et surtout conduire à des réactions indésirables de coupure ou de déshydrogénation de l'éther utilisé comme solvant.

La durée de la réaction, qui dépend de la température, de l'état de surface du métal et de la nature du milieu, sera suffisante pour convertir la proportion voulue d'hydrocarbure et pourra aller, par exemple, de 1/2 heure à 48 heures. Pendant la réaction le mélange sera avantageusement agité de façon mécanique ou par ultrasons.

La pression est sans effet important sur la réaction pourvu que les hydrocarbures insaturés et l'éther constituent une phase liquide. La réaction peut donc être effectuée à pression atmosphérique, mais si

nécessaire on peut opérer entre 1 kPa et 10 MPa.

Comme les composés formés sont très réactifs vis à vis de l'oxyène et des composés à protons actifs, la préparation de l'organolanthanide, la réaction et l'isolement des produits se font en atmosphère inerte, par exemple sous azote ou argon ou sous vide.

Les composés obtenus peuvent être débarassés du solvant par décantation ou évaporation. Ils peuvent être dissouts et recristallisés dans un milieu convenablement choisi.

Les composés organométalliques des terres rares objects de l'invention répondent à la formule $MR_x$ dans laquelle R est défini par les formules (1 à 6) et x est égal à 1,2 ou 3. Ils trouvent les mêmes applications que les organolanthanides antérieurement connus, mais leur réactivité est généralement supérieure. C'est ainsi que par action des dérivés protiques tels que l'eau, les alcools, les acides ils conduisent aux hydrocarbures contenant 2, et éventuellement 4, atomes d'hydrogène de plus que les hydrocarbures de départ, par exemple au dihydroanthracène par protolyse du complexe anthracénique. La deutérolyse conduit aux composés di- ou éventuellement tétra-deutériés. Ils réagissent avec les cétones, les aldéhydes, les esters, les anhydrides, les dérivés halogénés, les sulfates et les sulfures pour conduire aux produits d'addition qui par action d'agents électrophiles conduisent aux composés réduits correspondants. Ils peuvent être utilisés pour catalyser l'hydrogénation des oléfines et des dioléfines et leur polymérisation.

Par chauffage les organolanthanides de l'invention conduisent à la formation du métal correspondant sous forme très finement divisée et donc très réactive, par exemple vis-à-vis de l'hydrogène. A partir d'un mélange de composés de plusieurs métaux distincts, on peut obtenir des alliages finement divisés. Le chauffage peut être effectué dans un milieu hydrocarbure, par exemple dans le toluène bouillant.

Les exemples suivants illustrent l'invention sans en limiter la portée.

EXEMPLE N°1

Dans un tube de Schlenk, préalablement débarassé d'oxygène et d'humidité, on a introduit 20 m.atomes g d'ytterbium en poudre et 10 ml d'éther diméthylique de l'éthylène glycol (DME) contenant 280 mg de diiodo-1,2 éthane. On a laissé réagir avec agitation, à température ambiante, pendant une heure. On a alors refroidi la suspension à -20° C puis on a ajouté 5 m.moles d'anthracène dissous dans 35 ml de DME. On a laissé réagir pendant 20 heures à température ambiante. Il s'est formé, avec un rendement pratiquement quantitatif par rapport à l'anthracène, un précipité violet caractérisé comme étant l'anthracène-ytterbium (1.1) solvaté par du DME. Le complexe, traité par du méthanol deutérié, a conduit à du dideutéro-9,10 anthracène avec un rendement quantitatif (>99%).

EXEMPLE N°2

On a opéré comme dans l'exemple 1 mais en utilisant 20 m.atomes g de poudre de samarium à la place de l'ytterbium. On a obtenu de l'anthracène samarium (1/1), solvaté par du DME, sous forme d'un composé violet, avec un rendement pratiquement quantitatif par rapport à l'anthracène. Le complexe traité par un excès d'eau a conduit à du dihydroanthracène avec un rendement quantitatif.

EXEMPLE N°3

On a opéré comme dans l'exemple n°2 à ceci près qu'on a introduit 5 m.moles de diphényl-1,4 butadiène à la place de l'anthracène. On a obtenu un précipité marron-rouge caractérisé comme étant le diphénylbutadiène-samarium (1/1) solvaté par du DME avec un rendement de 43% par rapport à l'hydrocarbure de départ. Par action d'un excès d'iodure de méthyle on a obtenu un mélange d'isomères du diphényl-1,4 butènes diméthylés avec un rendement de 50 %.

EXEMPLE N°4

On a opéré comme dans l'exemple 1 à ceci près qu'on a remplacé l'anthracène par la quantité molaire équivalente de cyclooctatétraène. Le composé violet obtenu avec un rendement pratiquement quantitatif a été caractérisé comme étant le cyclooctatétraène-ytterbium solvaté par du DME.

EXEMPLE N°5

Dans un tube de Schlenk purgé de l'oxygène et de l'humidité on a introduit 19 m.atomes g de poudre d'ytterbium puis 10 ml de DME contenant 1 m.mole de diiodo-1,2 éthane. On a laissé réagir à température ambiante pendant 1 heure avec agitation, puis on a refroidi à -78° C et on a injecté 5 m.moles de butadiène-1,3 gazeux. On a maintenu le tube pendant 20 heures entre -78°C et -50°C. On a obtenu un précipité gris caractérisé comme étant le butadiène-ytterbium (1/1) solvaté par du DME avec un rendement de 95 % par rapport au butadiène. L'action d'un excès d'iodure de méthyle a conduit à des hexènes isomères avec en prédominance de l'hexène-3 et du méthyl-3 pentène 1.

EXEMPLE N°6

On a opéré comme dans l'exemple 1 à ceci près qu'on a introduit la quantité équivalente molaire de diphényl-1,4 butadiène à la place de l'anthracène. On a obtenu un précipité rouge foncé caractérisé comme étant le diphényl-1,4 butadiène-ytterbium (1/1) solvaté par du DME avec un rendement quantitatif. La deutérolyse de ce composé a conduit aux dideutérodiphénylbutène-1 et 2 avec un rendement supérieur à 99%.

EXEMPLE N°7

Dans un autoclave en acier inoxydable de 100 ml de volume, préalablement débarassé de traces d'oxygène et d'humidité, on a introduit 5,8 $10^{-4}$ mole du complexe anthracène samarium de l'exemple 2 en suspension dans 5 ml d'heptane. On a pressurisé l'autoclave par 4 MPa d'éthylène à 25°C puis on a introduit de l'hydrogène de façon à maintenir la pression totale à 5 MPa. On a mis en route l'agitation et après 15 minutes on a constaté que le gaz ne contenait plus d'éthylène qui avait été converti en éthane ; il s'était également formé du polyéthylène de haute masse.

EXEMPLE N°8

On a opéré comme dans l'exemple 7 à ceci près qu'on n'a pas introduit d'hydrogène. Après 30 minutes d'agitation on a ouvert l'autoclave qui contenait 4 g de polyéthylène de haute masse.

EXEMPLE N° 9 A 20

Le mode opératoire général utilisé dans ces exemples est le suivant : un tube de Schlenk muni d'un septum, purgé de toute trace d'oxygène et d'humidité et maintenu tout au long de la manipulation sous atmosphère d'argon, est chargé avec la quantité voulue de métal ; on y introduit ensuite 1,3 ml de diméthoxyéthane (DME) contenant 5 % molaire par rapport au métal de diiodo-1,2 éthane. Après avoir agité le milieu pendant deux heures à température ambiante on refroidit le tube à -20°C et on y ajoute la quantité choisie du substrat organique dissous dans du DME. Après une heure, on remonte la température jusqu'à l'ambiante et on poursuit l'agitation pendant vingt heures ; la solution ou la suspension du composé organométallique ainsi formé est ensuite traitée par le réactif approprié.

EXEMPLE N° 9

Avec le mode opératoire décrit ci-dessus on a fait réagir 3 m atomes de samarium en poudre avec 1,4 mmole de benzophénone dans un volume de 6,3 ml de DME. Il s'est développée une coloration rouge qui a viré au bleu très foncé puis au jaune marron, on a ainsi obtenu une solution de samarium-benzophénone. Après traitement par un excès de méthanol, évaporation du solvant, dissolution du métal dans de l'acide sulfurique dilué, extraction par de l'éther diéthylique on a receuilli quantitativement un produit qui a été caractérisé comme étant le benzhydrol.

EXEMPLE N° 10

On a opéré comme dans l'exemple 9 à ceci près qu'on a traité le complexe samarium-benzophénone par un excès de chlorure de benzyle. Après traitements on a isolé comme produit principal le diphénylbenzylcarbinol accompagné de 10 % de triphénylethylene.

EXEMPLE N° 11

On a opéré comme dans l'exemple 9 à ceci près qu'on a fait réagir 1,86 mmole de diterbuyl-2,6 anthracène sur 7,45 m atomes de samarium en poudre en soumettant le milieu à l'action d'ultrasons. On a obtenu ainsi une suspension violette visqueuse de diterbutyl-2,6 anthracène samarium en propositions équimolaires, qui a été traitée par 7,45 mmoles d'acétone. On a isolé du milieu du dihydro-9,10 diterbutyl-2,6 (9-isopropylhydroxy) anthracène.

EXEMPLE N° 12

Avec le même mode opératoire que celui de l'exemple 9 on a fait réagir 0,63 mmole de benzile sur 2,73 m atomes de samarium en poudre dans 7 ml de DME. La couleur a évolué du rouge au vert jaune puis au vert foncé. La solution obtenue renfermait le benzile-samarium. Le traitement par du méthanol a conduit à la formation de phénylacetophénone ainsi qu'à de plus faibles quantités de dihydrophénanthrène.

EXEMPLE N° 13

Avec le même mode opératoire que celui de l'exemple 9 on a fait réagir 0,77 mmole de chalcone sur 3,07 m atomes de samarium en poudre dans 6,2 ml de DME. La coloration du milieu a évolué du vert au marron puis au rouge-orangé, on a ainsi obtenu une solution de chalcone-samarium. Après l'action d'un excès de méthanol on a recueilli un mélange de diphényl-1,3 propanone-1, de diphyényl-1,3 propanol-1 et de diphényl-1,3 propène-2 ol-1.

EXEMPLE N° 14

Dans les conditions de l'exemple 9 on a fait réagir 1,5 mmole de dihexyl-1,8 anthracène sur 6,04 m atomes de samarium en poudre dans 11 ml de DME. On a obtenu une solution bleu foncé qui a été filtrée de l'excès de métal. L'évaporation du solvant a conduit à un composé dont l'analyse élémentaire correspond au composé dihexylanthracène samarium 1/1.

EXEMPLE N° 15

Dans les conditions de l'exemple 9 on a fait réagir 2,52 mmoles de benzile sur 9,84 m atomes de néodyme en poudre dans 15 ml de DME. La coloration du milieu à évolué du bleu au rouge puis au vert, on a ainsi obtenu

une solution de benzile-néodyme. Après action d'un excès de méthanol puis traitement habituel on a isolé de la phénylacétophénone (10 0/0), de la benzoïne (25 0/0) et du dihydro phénanthrène (1 0/0).

EXEMPLE N° 16

Dans les conditions de l'exemple 9 on a fait réagir 2,55 m atomes d'ytterbium sur 0,64 mmole de diméthyl-2,6 anthracène dans 8 ml de DME, et on a obtenu le diméthyl-2,6 anthracène ytterbium 1/1. Après action d'un excès de méthanol deutérié et traitement habituel on a isolé quantitativement du dideutéro-9,10 dihydro-9,10 diméthyl-2,6 anthracène.

EXEMPLE N° 17

Avec le même mode opératoire que celui de l'exemple 9 on a fait réagir 1,16 mmole de cyclooctatétraène sur 4,65 m atomes de cérium en poudre dans 6,5 ml de DME. La suspension grise de cyclooctatétraène cérium alors obtenue a été deutérolysée par du méthanol deutérié puis traitée comme ci-avant. On a obtenu quantitativement le dideutérocyclooctatriène sous la forme de trois isomères.

EXEMPLE N° 18

Dans les conditions de l'exemple 9 on a fait réagir 1,14 mmole d'anthracène avec 4,57 m atomes de cérium en poudre dans 7,5 ml de DME avec application d'ultrasons, on a obtenu l'anthracène cérium. Après action d'un excès de méthanol et traitement habituel on a isolé du dihydro-9,10 anthracène avec un rendement de 59 0/0.

EXEMPLE N° 19

Dans les conditions de l'exemple 9 on a fait réagir 1,38 mmole de diphényl-1,4 butadiène 1,3 sur 5,52 m atomes de cérium en poudre dans 8 ml de DME avec application temporaire d'ultrasons, on a ainsi obtenu une solution de diphényl-1,4 butadiène-1,3 cérium. L'action d'un excès de méthanol et le traitement habituel ont permis d'isoler des diphényl-1,4 butènes (trois isomères) avec un rendement de 29 0/0.

EXEMPLE N° 20

Dans les conditions de l'exemple 9 on a fait réagir 0,85 mmole de benzoate de phényle dans 6 ml de DME sur 3,38 m atomes de samarium. Le composé brun foncé qui a été obtenu (benzoate de phényle-samarium) a été traité de la façon habituelle. Le benzoate a été converti quantitativement.

**Revendications**

1 - Procédé d'obtention de composés organométalliques de lanthanides qui consiste à faire réagirm en milieu éther, un métal de la série des lanthanides sur au moins un composé organique insaturé renfermant au moins deux insaturations à une température de - 80 à + 100° C.

2 - Procédé selon la revendication 1 dans lequel le composé insaturé est choisi parmi les di-, tri-, tétraoléfines conjuguées des formules générales :

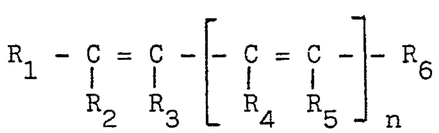 et 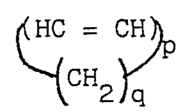

dans lesquelles $R_1$ à $R_6$ sont choisis parmi l'atome d'hydrogène et les restes hydrocarbyles, n étant un entier de 1 à 3, p un entier de 2 à 4 et 1 un entier de 1 à 5 ou zéro, la somme p + q étant au moins égale à 3.

3 - Procédé selon la revendication 1 dans lequel le composé insaturé est un hydrocarbure aromatique comportant au moins deux cycles condensés.

4.- Procédé selon la revendication 1 dans lequel le composé insaturé est une cétone ayant pour formule générale $RCOR^1$, $RCOXCOR^1$ ou

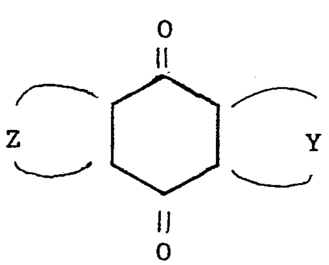

où R est un radical aryl, alkylaryl, alkényl ou cycloalkényl et $R^1$ est choisi parmi les restes hydrocarbyles comportant de 1 à 20 atomes de carbone, X est une liaison directe ou un reste alkylène ayant 2 à 10 atomes de carbone, Y et Z sont une liaison simple ou complètent un cycle aromatique.

5 - Procédé selon la revendication 1 dans lequel composé insaturé est un ester ayant pour formule ArCOOR où Ar est un radical aryl ou alkylaryl comportant de 6 à 20 atomes de carbone et R un radical hydrocarbyle comportant de 1 à 20 atomes de carbone.

6 - Procédé selon la revendication 1, dans lequel le composé insaturé est la quinoléine, l'isoquinoléine, l'acridine, la phénazine, la pyridazine, la phénanthroline, une azocine ou une base de Schiff.

7 - Procédé selon l'une des revendication 1 à 6, dans lequel le métal est le samarium, l'europium ou l'ytterbium.

8 - Procédé selon l'une des revendications 1 à 7, dans lequel l'éther est choisi parmi le diméthyléther, le tétrahydrofuranne, le diméthyléther de l'éthylène glycol, le diméthyléther du diéthylène glycol et les éthers cycliques de l'éthylène glycol comportant de 4 à 8 groups $-CH_2 CH_2 O-$.

9 - Procédé selon l'une des revendications 1 à 8, dans lequel le milieu éther renferme au moins un hydrocarbure du groupe des hydrocarbures saturés et des hydrocarbon aromatiques.

10 - Procédé selon l'une des revendications 1 à 9, dans lequel le métal est sous forme divisée activée par un halogénure d'hydrocarbyle, un halogène, un halogénure mercurique ou un halogénure de métal de terre rare.

11 - Les composés obtenus par la procédé selon l'une des revendications 1 à 10.

12 - Utilisation des composés obtenus selon le procédé de l'une des revendications 1 à 10 pour catalyser l'hydrogénation des oléfines ou des dioléfines.

13 - Utilisation des composés obtenus elon le procédé de l'une des revendications 1 à 10 pour catalyser la polymérisation ou la copolymérisation de l'éthylène.

14 - Utilisation des composés obtenus selon le procédé de l'une des revendications 1 à 10 pour produire par chauffage le lanthanide métal correspondant sous forme divisée.